# EUROPEAN PATENT APPLICATION

(11) **EP 4 316 265 A1**
(43) Date of publication of application: **07.02.2024**
(21) Application number: 22775250.8
(22) Date of filing: 15.03.2022
(51) Int. Cl.: A23L 2/52, A23L 33/105

(54) **COROSOLIC-ACID-CONTAINING BEVERAGE AND METHOD FOR MANUFACTURING SAME**

(30) Priority: 22.03.2021 JP 2021047118
(71) Applicant: Suntory Holdings Limited, Osaka 530-8203 (JP)
(72) Inventor: ITOGA, Shota, Soraku-gun, Kyoto 619-0284 (JP); FUKIZAWA, Shinya, Soraku-gun, Kyoto 619-0284 (JP); NONAKA, Yuji, Soraku-gun, Kyoto 619-0284 (JP)
(74) Representative: Vossius & Partner Patentanwälte Rechtsanwälte mbB
(86) International application number: PCT/JP2022/011455
(87) International publication number: WO 2022/202459

(57) **Abstract**

The present invention aims to provide a corosolic acid-containing beverage with an increased solubility of corosolic acid in water and a method of producing the same. The present invention relates to a corosolic acid-containing beverage that is a beverage containing corosolic acid, the beverage containing: at least one type of epicatechin compound selected from the group consisting of epicatechin, epigallocatechin, epicatechin gallate, and epigallocatechin gallate, wherein a corosolic acid content is 1 ppm or higher and corosolic acid is at least partially dissolved in the beverage.

## Description

### TECHNICAL FIELD

The present invention relates to a corosolic acid-containing beverage and a method of producing the same.

### BACKGROUND ART

Banabá (*Lagerstroemia speciosa*) is a plant belonging to the genus *Lagerstroemia* of the family Lythraceae, and its leaves have long been used to brew a health tea called "Banabá tea".

Compounds known as components of Banabá include corosolic acid (IUPAC name:
(1S,2R,4aS,6aR,6aS,6bR,8aR,10R,11R,12aR,14bS)-10,11-dihydroxy-1,2,6a,6b,9,9,12a-heptamethyl-2,3,4,5,6,6a,7,8,8a,10,11,12,13,14b-tetradecahydro-1H-picene-4a-carboxylic acid), a type of triterpene (Non-Patent Literature 1).

Corosolic acid is known to have an anti-cancer effect, an anti-hyperglycemic effect, an anti-obesity effect, an anti-inflammatory effect, an anti-hyperlipidemic effect, an anti-viral effect, and other various health benefits. The reported mechanisms of action include AMP-activated protein kinase (AMPK) activation, inhibition of nuclear factor-κB (NFκB) activation, inhibition of interleukin-1 receptor-associated kinase 1 (IRAK-1), and inhibition of reactive oxygen species (ROS) production (Non-Patent Literature 2). In recent years, there has been attention to the corosolic acid-induced vascular endothelial function normalization effect, and a corosolic acid-induced Tie-2 activation effect has also been reported (Patent Literature 1). Non-Patent Literature 3 has reported an effect of inhibiting elevation of blood glucose levels in human by ingestion of a corosolic acid-containing extract over one to two weeks.

Since corosolic acid has various effects as described above, provision of functional beverages and functional foods containing corosolic acid seemingly contributes to maintenance and enhancement of health.

Corosolic acid, however, has a low solubility in water, so that even Banabá tea extracted with hot water from Banabá leaves contains only a trace amount of corosolic acid. Thus, in the case of preparing a functional material containing corosolic acid, corosolic acid is possibly extracted from plants or other materials using an organic solvent containing ethanol or the like. Patent Literature 2 discloses a method of producing a corosolic acid-rich extract by extracting corosolic acid with an aqueous ethanol solution and then treating the corosolic acid with activated charcoal.

### CITATION LIST

### - Patent Literature

Patent Literature 1: JP 2014-97977 A
Patent Literature 2: JP 2005-263650 A
Patent Literature 3: JP 2013-13392 A

### - Non-Patent Literature

Non-Patent Literature 1: Diabetes Res Clin Pract. 2006 Aug; 73(2): 174-7
Non-Patent Literature 2: Oncol Lett. 2021 Feb; 21(2): 84
Non-Patent Literature 3: Journal for the Integrated Study of Dietary Habits, 2006, vol. 17, No. 3, pp. 255-259

### SUMMARY OF INVENTION

### - Technical Problem

Since corosolic acid has a low solubility in water, production of a beverage with corosolic acid dissolved is difficult. For example, corosolic acid is ingested in the form of a capsule in Non-Patent Literature 3 presumably because corosolic acid is difficult to provide in the form of a beverage. Meanwhile, what is important in providing functional foods and functional beverages is to provide the foods and beverages in a form that is easy to ingest in an ongoing basis. One such form can be beverages. Still, in the case of an active ingredient having a low solubility in water, the active ingredient in the beverage may precipitate to be left in the container in a considerable amount even when the beverage is drunk up. Thus, even when the active ingredient having a low solubility in water is blended in a beverage in an amount enough to achieve the desired effect (such an amount is also referred to as an effective amount), the effective amount of the active ingredient may be difficult to ingest without fail. Another possible technique to employ is increasing the solubility of the active ingredient in water to enhance the intestinal absorption of the active ingredient such that the desired functions can be sufficiently demonstrated. In response to these issues, a technique of increasing the solubility of corosolic acid in water is demanded.

There are techniques to increase the solubility of a component having a low solubility in water. For example, Patent Literature 3 discloses a technique of increasing the solubility of a hardly water-soluble polyphenol by subjecting the hardly water-soluble polyphenol together with one or two or more selected from the catechins, chlorogenic acids, and methylated compounds of hardly water-soluble polyphenols to a heat treatment at from 110°C to 180°C. Corosolic acid, however, is a compound classified as a triterpene, which has different properties from polyphenols. Although there are techniques to increase the solubility of a component having a low solubility in water as described above, no specific method of increasing the solubility of corosolic acid has been considered.

The present invention aims to provide a corosolic acid-containing beverage with an increased solubility of corosolic acid in water and a method of producing the same.

### - Solution to Problem

As a result of intensive studies to solve the problem, the present inventors found that the solubility of corosolic acid in water increases in a solution containing at least one type of epicatechin compound selected from the group consisting of epicatechin, epicatechin gallate, epigallocatechin, and epigallocatechin gallate. It has been difficult to infer by analogy the increase in solubility of corosolic acid in water in the presence of an epicatechin compound based on the feature of the chemical structural formula of corosolic acid.

In other words, the present invention relates to, but is not limited to, the following corosolic acid-containing beverages and methods of producing the same.
(1) A corosolic acid-containing beverage that is a beverage containing corosolic acid, the beverage containing: at least one type of epicatechin compound selected from the group consisting of epicatechin, epigallocatechin, epicatechin gallate, and epigallocatechin gallate, wherein a corosolic acid content is 1 ppm or higher and corosolic acid is at least partially dissolved in the beverage.
(2) The corosolic acid-containing beverage according to (1) above, wherein a total concentration of epicatechin, epigallocatechin, epicatechin gallate, and epigallocatechin gallate is from 1.7 to 1700 ppm.
(3) The corosolic acid-containing beverage according to (1) or (2) above, wherein the corosolic acid-containing beverage is a beverage in a sealed container.
(4) The corosolic acid-containing beverage according to (3) above, wherein the container is a plastic bottle, a can, a bottle, or a retort pouch.
(5) The corosolic acid-containing beverage according to (3) or (4) above, wherein the container is a plastic bottle.
(6) A method of producing a corosolic acid-containing beverage that is a beverage containing corosolic acid, the method including: a beverage preparation step of mixing corosolic acid with water in the presence of at least one type of epicatechin compound selected from the group consisting of epicatechin, epigallocatechin, epicatechin gallate, and epigallocatechin gallate, and thereby obtaining a beverage containing the at least one type of epicatechin compound, corosolic acid, and water, with corosolic acid being at least partially dissolved, the beverage preparation step including mixing corosolic acid with water to a corosolic acid content of 1 ppm or higher in the beverage.
(7) The production method according to (6) above, wherein in the beverage preparation step, the at least one type of epicatechin compound is present in an amount that results in a total concentration of epicatechin, epigallocatechin, epicatechin gallate, and epigallocatechin gallate in the range of 1.7 to 1700 ppm in the beverage.

### - Advantageous Effects of Invention

The present invention can provide a corosolic acid-containing beverage with an increased solubility of corosolic acid in water and a method of producing the same.

### DESCRIPTION OF EMBODIMENTS

### <Corosolic acid-containing beverage>

A beverage of the present invention is a beverage that contains corosolic acid. The beverage of the present invention is a corosolic acid-containing beverage containing at least one type of epicatechin compound selected from the group consisting of epicatechin, epigallocatechin, epicatechin gallate, and epigallocatechin gallate, wherein a corosolic acid content is 1 ppm or higher and corosolic acid is at least partially dissolved in the beverage.

The "ppm" herein means ppm by weight/volume (w/v). 1 ppm can be converted to 1 mg/L.

Corosolic acid is one of triterpene compounds and is classified as a ursane-type triterpene. In the present invention, the origin and production method of corosolic acid are not limited. Corosolic acid may be a synthetic product or an extract from a plant containing corosolic acid. Examples of the plant containing corosolic acid include Banabá (*Lagerstroemia speciosa*) belonging to the genus *Lagerstroemia* of the family Lythraceae. In one embodiment, corosolic acid may be an extract from Banabá leaves. Corosolic acid may also be a commercially available product.

A corosolic acid content in the corosolic acid-containing beverage is 1 ppm or higher. In the beverage of the present invention, corosolic acid in the beverage is at least partially dissolved in the beverage.

The corosolic acid content in the beverage herein is the sum (ppm) of the amount of corosolic acid dissolved in the beverage and the amount of corosolic acid not dissolved in (precipitated from) the beverage.

In one embodiment, the corosolic acid content in the beverage is preferably 2 ppm or higher. In one embodiment, the upper limit of the corosolic acid content in the beverage is not limited and may be, for example, 10 ppm or lower. In one embodiment, the corosolic acid content in the beverage is preferably from 1 to 10 ppm, more preferably from 2 to 10 ppm.

The corosolic acid content is measurable, for example, with a liquid chromatography-tandem mass spectrometry (LC-MS/MS) device by liquid chromatography-mass spectrometry. The measurement conditions can be the conditions described in EXAMPLES, for example.

A beverage containing 1 ppm or higher corosolic acid and an epicatechin compound dissolves an increased amount of corosolic acid compared to a beverage containing 1 ppm or higher corosolic acid but no epicatechin compound. Use of an epicatechin compound can increase the solubility of corosolic acid in water.

In the present invention, corosolic acid in the beverage is at partially dissolved in the beverage and may be entirely dissolved in the beverage. The amount (concentration) of the compound dissolved is measurable by, for example, a method including sufficiently mixing the compound to be measured with water to prepare an aqueous solution (or a saturated aqueous solution), removing insoluble matter by centrifugation, and measuring the concentration in the supernatant. In one embodiment, the proportion of corosolic acid dissolved in the beverage relative to corosolic acid present in the beverage is preferably 30 wt% or more, more preferably 35 wt% or more, still more preferably 40 wt% or more. In one embodiment, the proportion of corosolic acid dissolved in the beverage relative to corosolic acid present in the beverage may be 100 wt% or less, or may be 95 wt% or less.

In one embodiment, the concentration of corosolic acid dissolved in the beverage is preferably 0.3 ppm or higher, more preferably 0.4 ppm or higher, still more preferably 0.45 ppm or higher. In one embodiment, the concentration of corosolic acid dissolved in the beverage may be 10 ppm or lower.

The corosolic acid-containing beverage contains at least one type of epicatechin compound selected from the group consisting of epicatechin, epigallocatechin, epicatechin gallate, and epigallocatechin gallate. The corosolic acid-containing beverage contains at least one of the four types, namely epicatechin, epigallocatechin, epicatechin gallate, and epigallocatechin gallate, and may contain two or more of the four types. In one embodiment, the corosolic acid-containing beverage preferably contains epicatechin, epigallocatechin, epicatechin gallate, and epigallocatechin gallate.

The origin and production method of epicatechin compounds are not limited. The epicatechin compounds may each be a synthetic product or a plant-derived extract such as a green tea extract. For example, the green tea extract typically contains the four types of epicatechin compounds above. In the case of a plant-derived extract, epicatechin compounds may be added as tea extracts.

The total concentration of epicatechin, epigallocatechin, epicatechin gallate, and epigallocatechin gallate (hereinafter, also referred to as a total epicatechin compound concentration) in the beverage is preferably 1.7 ppm or higher. With a total epicatechin compound concentration of 1.7 ppm or higher, the solubility of corosolic acid in water can be increased. The upper limit of the total epicatechin compound concentration in the corosolic acid-containing beverage is not limited. In one embodiment, in terms of flavor, the total epicatechin compound concentration in the corosolic acid-containing beverage is preferably 1700 ppm or lower.

In one embodiment, the total epicatechin compound concentration in the beverage is more preferably 17 ppm or higher, while it is more preferably 1360 ppm or lower, still more preferably 340 ppm or lower. In one embodiment, the total concentration of epicatechin, epigallocatechin, epicatechin gallate, and epigallocatechin gallate in the beverage is preferably from 1.7 to 1700 ppm, more preferably from 1.7 to 1360 ppm, still more preferably from 1.7 to 340 ppm, particularly preferably from 17 to 340 ppm.

The epicatechin compound concentration is measurable by high-performance liquid chromatography (HPLC). Specifically, with epicatechin (EC), epigallocatechin (EGC), epicatechin gallate (ECG), or epigallocatechin gallate (EGCG) used as a standard, the concentrations of epicatechin, epigallocatechin, epicatechin gallate, and epigallocatechin gallate are measured by high-performance liquid chromatography (HPLC), so that the sum of the concentrations of these four types can be calculated as the total epicatechin compound concentration.

In one embodiment, preferred ranges of the corosolic acid content and the total epicatechin compound concentration in the beverage are as follows, for example.

In one embodiment, if the corosolic acid content in the beverage is from 1 to 5 ppm, the total epicatechin compound concentration is preferably from 1.7 to 1700 ppm, more preferably from 1.7 to 1360 ppm, still more preferably from 1.7 to 680 ppm, even more preferably from 1.7 to 340 ppm, particularly preferably from 17 to 340 ppm.

In one embodiment, if the corosolic acid content in the beverage is higher than 5 ppm and 10 ppm or lower, the total epicatechin compound concentration is preferably from 1.7 to 340 ppm, more preferably from 17 to 340 ppm.

With the corosolic acid content and the total epicatechin compound concentration falling within the respective ranges above, the solubility of corosolic acid in water is further increased compared to when no epicatechin compound is contained.

In another embodiment, for example, if the corosolic acid content in the beverage is from 1 to 2 ppm, the total epicatechin compound concentration may be from 340 to 1700 ppm, may be higher than 340 ppm and 1700 ppm or lower, or may be from 500 to 1700 ppm.

The pH of the corosolic acid-containing beverage is not limited. The pH is preferably 7.4 or lower, more preferably from 3.0 to 6.5. The pH herein means a pH at 25°C. The pH is measurable with a commercially available pH meter.

The corosolic acid-containing beverage of the present invention contains water. The corosolic acid-containing beverage of the present invention is usually a water-based liquid composition and has a water content of preferably 90 wt% or higher, more preferably 95 wt% or higher. The water content may be, for example, 99.9 wt% or lower. The corosolic acid-containing beverage may or may not contain ethanol. In one embodiment, the corosolic acid-containing beverage has an ethanol concentration of preferably lower than 5 v/v%, more preferably 1 v/v% or lower, still more preferably lower than 1 v/v%. According to the present invention, in a beverage with an ethanol concentration of lower than 1 v/v%, the solubility of corosolic acid can be increased.

The corosolic acid-containing beverage may contain at least one type of catechin compound selected from the group consisting of catechin, gallocatechin, catechin gallate, and gallocatechin gallate, and may contain these four types of chatechin compounds.

The corosolic acid-containing beverage may be, for example, a tea-based beverage, a coffee beverage, an alcoholic beverage, or a functional beverage. In one embodiment, the beverage of the present invention is preferably a tea-based beverage. Examples of the tea-based beverage include green tea, black tea, oolong tea, *Pu'er* tea, blended tea, and herbal tea. In particular, the corosolic acid-containing beverage is preferably a green tea beverage. The green tea beverage is a beverage obtained by blending green tea extracts from green tea leaves, and is preferably a beverage that contains the green tea extracts as a principal component other than water.

The corosolic acid-containing beverage may contain one or two or more types of additives such as flavors, vitamins, dyes, antioxidants, sourness agents, emulsifiers, preservatives, seasonings, extracts, pH adjusters, and quality stabilizers, as long as they do not inhibit the effect of the present invention.

For ease of transport and long-term shelf life, the beverage of the present invention is preferably a beverage in a sealed container in a state where the beverage is sealed in a container (corosolic acid-containing beverage in a sealed container). The corosolic acid-containing beverage in a sealed container is obtained by filling the container with the corosolic acid-containing beverage and the container is usually sealed. The container for the beverage in a sealed container is not limited and is preferably a plastic bottle, a can, a bottle, or a retort pouch, more preferably a plastic bottle.

In one embodiment, the corosolic acid-containing beverage of the present invention is preferably a beverage in a sealed container obtained by filling a plastic bottle with a corosolic acid-containing beverage.

The method of producing the beverage of the present invention is not limited. For example, the following production method can be employed.

### <Method of producing corosolic acid-containing beverage>

The present invention encompasses the following method of producing a corosolic acid-containing beverage.

A method of producing a corosolic acid-containing beverage that is a beverage containing corosolic acid, the method including: a beverage preparation step of mixing corosolic acid with water in the presence of at least one type of epicatechin compound selected from the group consisting of epicatechin, epigallocatechin, epicatechin gallate, and epigallocatechin gallate, and thereby obtaining a beverage containing the at least one type of epicatechin compound, corosolic acid, and water, with corosolic acid being at least partially dissolved, the beverage preparation step including mixing corosolic acid with water to a corosolic acid content of 1 ppm or higher in the beverage

The production method above enables production of a corosolic acid-containing beverage in which a corosolic acid content is 1 ppm or higher and corosolic acid is at least partially dissolved.

In the production method of the present invention, the corosolic acid-containing beverage and preferred embodiments thereof are the same as those for the corosolic acid-containing beverage of the present invention above.

Mixing corosolic acid with water in the presence of the epicatechin compound(s) can increase the solubility of corosolic acid. Thereby, a beverage (composition) can be prepared that contains at least one type of epicatechin compound, corosolic acid, and water, with corosolic acid being at least partially dissolved.

In the beverage preparation step, the epicatechin compound(s), water, and corosolic acid are mixed. As long as the effect of the present invention is not impaired, components that can be blended in a beverage other than the epicatechin compound(s), water, and corosolic acid may be added and mixed. In the beverage preparation step, the order of mixing the components is not limited. For example, the epicatechin compound(s) may be mixed with water first and the resulting mixture may be mixed with corosolic acid, corosolic acid may be mixed with water first and the resulting mixture may be mixed with the epicatechin compound(s), or the epicatechin compound(s) and corosolic acid may be mixed with water. Also, for example, corosolic acid and other components may be added to a tea beverage (preferably a green tea beverage) containing epicatechin compound(s) to prepare a corosolic acid-containing beverage.

In the beverage preparation step, water is mixed with corosolic acid in an amount that results in a corosolic acid content of 1 ppm or higher in the beverage to be obtained. In one embodiment, water is mixed with corosolic acid in an amount that results in a corosolic acid content of preferably 1 ppm or higher, more preferably 2 ppm or higher, but preferably 10 ppm or lower, in the beverage.

In the beverage preparation step, the epicatechin compound(s) is/are preferably used in an amount that results in a total concentration of epicatechin, epigallocatechin, epicatechin gallate, and epigallocatechin gallate of from 1.7 to 1700 ppm in the beverage to be obtained. In the beverage preparation step, the solubility of corosolic acid can be increased in the presence of epicatechin compound(s) that results in a total concentration of epicatechin, epigallocatechin, epicatechin gallate, and epigallocatechin gallate in the range of 1.7 to 1700 ppm in the beverage. In one embodiment, in the beverage preparation step, epicatechin compound(s) is/are used in an amount that results in a total concentration of epicatechin, epigallocatechin, epicatechin gallate, and epigallocatechin gallate in the range of more preferably 1.7 to 1360 ppm, still more preferably 1.7 to 680 ppm, particularly preferably 17 to 340 ppm, in the beverage.

The preferred ranges of the corosolic acid content/concentration and the epicatechin compound content/concentration in the beverage to be obtained in the beverage preparation step and other conditions are the same as those for the corosolic acid-containing beverage above.

In one embodiment, in the beverage preparation step, if water is mixed with corosolic acid in an amount that results in a corosolic acid content of from 1 to 2 ppm in the beverage, epicatechin compound(s) may be present in an amount that results in a total concentration of epicatechin, epigallocatechin, epicatechin gallate, and epigallocatechin gallate of from 340 to 1700 ppm, of higher than 340 ppm to 1700 ppm or lower, or in the range of 500 to 1700 ppm, in the beverage.

The temperature during the beverage preparation step is not limited. For example, the temperature can be from 4°C to 40°C, preferably from 10°C to 30°C.

The production method of the present invention may include a step of filling a container with a corosolic acid-containing beverage. The method of filling a container with a corosolic acid-containing beverage is not limited and can be selected according to the container and other conditions. For example, hot-pack filling or aseptic filling can be employed. The filling conditions can be set appropriately according to the type of container and other factors. Sealing the container after filling enables production of a corosolic acid-containing beverage in a sealed container. After filling with the beverage, a step of filling the headspace with nitrogen gas or carbon dioxide gas may be performed. The corosolic acid-containing beverage may be heat sterilized before filling the container, or may be heat sterilized after filling the container. In the case of performing heat sterilization, the method is not limited and may be a typical method.

The numerical range represented using a lower limit value and an upper limit value herein, i.e., "from a lower limit value to an upper limit value", is inclusive of the lower limit value and the upper limit value. For example, the range represented by "from 1 to 2" means from 1 to 2, inclusive of 1 and 2. Herein, the range may be any combination of any upper limit and any lower limit.

### EXAMPLES

Hereinafter, examples that more specifically describe the details of the present invention are shown. The present invention is not limited to these examples.

Centrifugation is typically used to separate substances that are not dissolved in a solution.

In the examples and comparative examples, the solubility of corosolic acid in water was evaluated by adding a certain amount of corosolic acid to water, mixing the mixture, centrifuging the resulting solution, and measuring the corosolic acid concentration in the supernatant.

The corosolic acid concentration in the supernatant was analyzed using a liquid chromatography-tandem mass spectrometry (LC-MS/MS) device. The LC-MS/MS conditions are as follows.

### <LC-MS/MS analysis conditions>

### [HPLC conditions]

Device: Ultimate 3000 (Thermo Fisher Scientific K.K.)
Column: Atlantis T3, 2.1 mm × 150 mm, particle size 3 um

### (Waters Corporation)

Mobile phase A: 0.1% formic acid aqueous solution
Mobile phase B: 0.1% formic acid acetonitrile
Injection amount: 4 µL
Flow rate: 0.250 mL/min
Column temperature: 40°C

### Gradient program

Table 1 shows the gradient program. The concentration (%) of each mobile phase is expressed as v/v%.

**[Table 1]**

| Time (min) | Phase A (%) | Phase B (%) |
|---|---|---|
| 0 | 75 | 25 |
| 2 | 75 | 25 |
| 10 | 0 | 100 |
| 20 | 0 | 100 |
| 25 | 75 | 25 |
| 26 | 75 | 25 |

### [MS/MS conditions]

Device: TSQ QUANTIVIA (product name, Thermo Fisher Scientific K.K.)
Measurement mode: MRM negative
Set mass-to-charge ratio (m/z): 471.3 → 423.3

In the following examples and comparative examples, a catechin mixture (available from Nagara Science Co., Ltd.) was used as a mixture of four types of compounds, namely epicatechin, epicatechin gallate, epigallocatechin, and epigallocatechin gallate (hereinafter, the compounds are also referred to as epicatechins). Corosolic acid (available from Nagara Science Co., Ltd.) was used as corosolic acid. The operations were performed at room temperature (from 20°C to 25°C) unless otherwise specified.

### <Examples 1 to 8>

First, the epicatechins were diluted with ultrapure water (Milli-Q, pH 7.4) to total concentrations (of the four types above) of from 1.7 to 340 ppm (hereinafter, the resulting dilutions are referred to as epicatechin solutions).

Next, corosolic acid was added to the epicatechin solutions such that the corosolic acid content would be 1 ppm (Examples 1 to 4) or 2 ppm (Examples 5 to 8). Thereby, corosolic acid-containing beverages were obtained.

Each of the obtained beverages was homogenized by thorough mixing, and then transferred to a 1.5-mL Eppendorf tube.

A centrifuge (available from Tomy Seiko Co., Ltd.) was used to centrifuge each beverage at 13,000 G for five minutes. Thereafter, the supernatant was collected and diluted two-fold with 100% ethanol. The resulting dilution was analyzed using a liquid chromatography-tandem mass spectrometry (LC-MS/MS) device, so that the concentration in the supernatant was calculated from the peak area of corosolic acid.

### <Comparative Examples 1 and 2>

Corosolic acid in an amount of giving a corosolic acid content of 1 ppm or 2 ppm was added to ultrapure water (Milli-Q) to obtain corosolic acid-containing beverages. Each of the obtained beverages was homogenized by thorough mixing, and then transferred to a 1.5-mL Eppendorf tube. The same procedure as in Example 1 was employed for centrifugation, supernatant collection, and analysis using a LC-MS/MS device, so that the corosolic acid concentration in the supernatant was calculated.

The relative ratio of the corosolic acid concentration in the supernatant of the beverage prepared in each of Examples 1 to 8 was determined by taking as 1 the corosolic acid concentration detected in the supernatant of the beverage (Comparative Example 1 or 2) with the same amount of corosolic acid added and 0 ppm epicatechins. Tables 2 shows the results of Examples 1 to 8 and Comparative Examples 1 and 2. In Table 2, the relative ratios of the concentrations in the supernatants in Examples 1 to 4 are values relative to the corosolic acid concentration in the supernatant of the beverage of Comparative Example 1. The relative ratios of the concentrations in the supernatants in Examples 5 to 8 are values relative to the corosolic acid concentration in the supernatant of the beverage of Comparative Example 2.

**[Table 2]**

| | Amount of epicatechins added (ppm) | Amount of corosolic acid added (ppm) | Relative ratio of concentration in supernatant |
|---|---|---|---|
| Comparative Example 1 | 0 | 1 | 1.00 |
| Example 1 | 1.7 | 1 | 2.15 |
| Example 2 | 17 | 1 | 1.92 |
| Example 3 | 170 | 1 | 2.54 |
| Example 4 | 340 | 1 | 2.80 |
| Comparative Example 2 | 0 | 2 | 1.00 |
| Example 5 | 1.7 | 2 | 1.11 |
| Example 6 | 17 | 2 | 1.34 |
| Example 7 | 170 | 2 | 1.68 |
| Example 8 | 340 | 2 | 1.77 |

### <Examples 9 to 14>

The epicatechins were diluted with ultrapure water (Milli-Q) to total concentrations of from 1.7 to 340 ppm, whereby epicatechin solutions were obtained. Next, corosolic acid was added to the epicatechin solutions such that the corosolic acid content would be 5 ppm (Examples 9 to 12) or 10 ppm (Examples 13 and 14). Thereby, corosolic acid-containing beverages were obtained. Each of the obtained beverages was homogenized by thorough mixing, and then transferred to a 1.5-mL Eppendorf tube.

The same procedure as in Example 1 was employed for centrifugation, supernatant collection, and analysis using a LC-MS/MS device, so that the corosolic acid concentration in the supernatant was calculated.

### <Comparative Examples 3 and 4>

Corosolic acid was added to ultrapure water (Milli-Q) to a corosolic acid content of 5 ppm or 10 ppm to obtain corosolic acid-containing beverages. Each of the obtained beverages was homogenized by thorough mixing, and then transferred to a 1.5-mL Eppendorf tube. The same procedure as in Example 1 was employed for centrifugation, supernatant collection, and analysis using a LC-MS/MS device, so that the corosolic acid concentration in the supernatant was calculated.

The relative ratio of the corosolic acid concentration in the supernatant of the beverage prepared in each of Examples 9 to 14 was determined by taking as 1 the corosolic acid concentration detected in the supernatant of the beverage (Comparative Example 3 or 4) with the same amount of corosolic acid added and with 0 ppm epicatechins. Table 3 shows the results of Examples 9 to 14 and Comparative Examples 3 and 4. In Table 3, the relative ratios of the concentrations in the supernatants in Examples 9 to 12 are values relative to the corosolic acid concentration in the supernatant of the beverage of Comparative Example 3. The relative ratios of the concentrations in the supernatants in Examples 13 and 14 are values relative to the corosolic acid concentration in the supernatant of the beverage of Comparative Example 4.

**[Table 3]**

| | Amount of epicatechins added (ppm) | Amount of corosolic acid added (ppm) | Relative ratio of concentration in supernatant |
|---|---|---|---|
| Comparative Example 3 | 0 | 5 | 1.00 |
| Example 9 | 1.7 | 5 | 1.03 |
| Example 10 | 17 | 5 | 1.15 |
| Example 11 | 170 | 5 | 1.13 |
| Example 12 | 340 | 5 | 1.22 |
| Comparative Example 4 | 0 | 10 | 1.00 |
| Example 13 | 1.7 | 10 | 1.07 |
| Example 14 | 17 | 10 | 1.04 |

### <Examples 15 to 20>

The epicatechins were diluted with ultrapure water (Milli-Q) to total concentrations of from 680 to 1700 ppm to obtain epicatechin solutions. Next, corosolic acid was added to the epicatechin solutions such that the corosolic acid content would be 1 ppm (Examples 15 to 17) or 2 ppm (Examples 18 to 20). Thereby, corosolic acid-containing beverages were obtained. Each of the obtained beverages was homogenized by thorough mixing, and then transferred to a 1.5-mL Eppendorf tube. The same procedure as in Example 1 was employed for centrifugation, supernatant collection, and analysis using a LC-MS/MS device, so that the corosolic acid concentration in the supernatant was calculated.

### <Comparative Examples 5 and 6>

Corosolic acid was added to ultrapure water (Milli-Q) to a corosolic acid content of 1 ppm or 2 ppm to obtain corosolic acid-containing beverages. Each of the obtained beverages was homogenized by thorough mixing, and then transferred to a 1.5-mL Eppendorf tube. The same procedure as in Example 1 was employed for centrifugation, supernatant collection, and analysis using a LC-MS/MS device, so that the corosolic acid concentration in the supernatant was calculated.

The relative ratio of the corosolic acid concentration in the supernatant of the beverage prepared in each of Examples 15 to 20 was determined by taking as 1 the corosolic acid concentration detected in the supernatant of the beverage (Comparative Example 5 or 6) with the same amount of corosolic acid added and with 0 ppm epicatechins. Table 4 shows the results of Examples 15 to 20. In Table 4, the relative ratios of the concentrations in the supernatants in Examples 15 to 17 are values relative to the corosolic acid concentration in the supernatant of the beverage of Comparative Example 5. The relative ratios of the concentrations in the supernatants in Examples 18 to 20 are values relative to the corosolic acid concentration in the supernatant of the beverage of Comparative Example 6.

**[Table 4]**

| | Amount of epicatechins added (ppm) | Amount of corosolic acid added (ppm) | Relative ratio of concentration in supernatant |
|---|---|---|---|
| Comparative Example 5 | 0 | 1 | 1.00 |
| Example 15 | 680 | 1 | 1.50 |
| Example 16 | 1360 | 1 | 1.57 |
| Example 17 | 1700 | 1 | 1.75 |
| Comparative Examle 6 | 0 | 2 | 1.00 |
| Example 18 | 680 | 2 | 1.06 |
| Example 19 | 1360 | 2 | 1.18 |
| Example 20 | 1700 | 2 | 1.14 |

An example where the relative ratio of the corosolic acid concentration (the relative ratio of the concentration in the supernatant) exceeds 1 can be regarded as a case where the solubility of corosolic acid in water increased owing to the co-presence of the epicatechins. The results above demonstrate that the solubility of corosolic acid in water further increased when corosolic acid was dissolved in an aqueous solution containing the epicatechin compounds compared to when corosolic acid was dissolved in water containing no epicatechin compound.

The corosolic acid concentration in the supernatant of the corosolic acid-containing beverage prepared in each of Comparative Examples 1 and 5 was 0.26 ppm. Among the corosolic acid-containing beverages of Examples 1 to 20 and Comparative Examples 1 to 6, the corosolic acid-containing beverages of Comparative Examples 1 and 5 were the lowest in terms of the corosolic acid concentration (0.26 ppm) in the supernatant. Among the corosolic acid-containing beverages of Examples 1 to 20 and Comparative Examples 1 to 6, the corosolic acid-containing beverages of Comparative Examples 1 and 5 were also the lowest in terms of the proportion of corosolic acid in the supernatant (the proportion (wt%) of corosolic acid dissolved in water) relative to corosolic acid added (100 wt%). In the corosolic acid-containing beverages prepared in Examples 1 to 20, the proportion of corosolic acid in the supernatant relative to corosolic acid added (100 wt%) was 40 wt% or higher.

### INDUSTRIAL APPLICABILITY

The present invention is useful in the food and beverage fields, for example.

## Claims

1. A corosolic acid-containing beverage that is a beverage containing corosolic acid, the beverage comprising:
at least one type of epicatechin compound selected from the group consisting of epicatechin, epigallocatechin, epicatechin gallate, and epigallocatechin gallate,
wherein a corosolic acid content is 1 ppm or higher and corosolic acid is at least partially dissolved in the beverage.

2. The corosolic acid-containing beverage according to claim 1,
wherein a total concentration of epicatechin, epigallocatechin, epicatechin gallate, and epigallocatechin gallate is from 1.7 to 1700 ppm.

3. The corosolic acid-containing beverage according to claim 1 or 2,
wherein the corosolic acid-containing beverage is a beverage in a sealed container.

4. The corosolic acid-containing beverage according to claim 3,
wherein the container is a plastic bottle, a can, a bottle, or a retort pouch.

5. The corosolic acid-containing beverage according to claim 3 or 4,
wherein the container is a plastic bottle.

6. A method of producing a corosolic acid-containing beverage that is a beverage containing corosolic acid, the method comprising:
a beverage preparation step of mixing corosolic acid with water in the presence of at least one type of epicatechin compound selected from the group consisting of epicatechin, epigallocatechin, epicatechin gallate, and epigallocatechin gallate, and thereby obtaining a beverage containing the at least one type of epicatechin compound, corosolic acid, and water, with corosolic acid being at least partially dissolved,
the beverage preparation step comprising mixing corosolic acid with water to a corosolic acid content of 1 ppm or higher in the beverage.

7. The production method according to claim 6,
wherein in the beverage preparation step, the at least one type of epicatechin compound is present in an amount that results in a total concentration of epicatechin, epigallocatechin, epicatechin gallate, and epigallocatechin gallate in the range of 1.7 to 1700 ppm in the beverage.
